Europäisches Patentamt

European Patent Office

Office européen des brevets

(19)

(11) Veröffentlichungsnummer: **0 035 584**
**B1**

(12) **EUROPÄISCHE PATENTSCHRIFT**

(45) Veröffentlichungstag der Patentschrift:
20.06.84

(51) Int. Cl.³: **A 61 F 13/00**

(21) Anmeldenummer: **80101234.5**

(22) Anmeldetag: **11.03.80**

(54) Ein Kissen enthaltender Druckverband.

(43) Veröffentlichungstag der Anmeldung:
**16.09.81 Patentblatt 81/37**

(45) Bekanntmachung des Hinweises auf die Patenterteilung:
**20.06.84 Patentblatt 84/25**

(84) Benannte Vertragsstaaten:
**AT BE CH FR GB IT NL SE**

(56) Entgegenhaltungen:
**DE - A - 2 124 803**
**DE - A - 2 213 182**
**DE - A - 2 722 563**
**DE - A - 2 810 737**
**DE - B - 1 491 215**
**DE - U - 1 895 718**
**DE - U - 7 410 200**
**DE - U - 7 806 873**
**US - A - 1 320 105**

**F. SMITH "Plastic and Reconstructive Surgery" 1950,**
**W.B. SAUNDERS CO., Philadelphia & London**

(73) Patentinhaber: **Schmid, Eduard, Dr.Dr.med.,**
**Böheimstrasse 37, D-7000 Stuttgart 1 (DE)**

(72) Erfinder: **Schmid, Eduard, Dr.Dr.med.,**
**Böheimstrasse 37, D-7000 Stuttgart 1 (DE)**

(74) Vertreter: **Patentanwälte Kohler - Schwindling - Späth,**
**Hohentwielstrasse 41, D-7000 Stuttgart 1 (DE)**

ACTORUM AG

Die Erfindung bezieht sich auf einen ein Kissen enthaltenden Druckverband zum Andrücken von in Körpern eingesetzten Hauttransplantaten, bei dem das Kissen mit einem Druckmedium gefüllt ist, Mittel zum Überwachen des Druckes im Kissen anschliessbar sind and über dem Kissen ein den Druck ausübender Teil des Verbandes angeordnet ist, zum Beispiel eine Binde gewickelt ist.

Bei der Transplantation von Haut, insbesondere dann, wenn die Haut in ihrer ganzen Dicke verpflanzt wird, ist es entscheidend, dass das ganze Transplantat mit einem gleichmässigen Druck auf die betreffende Körperstelle aufgedrückt wird. Dieser gleichmässige Druck muss sechs bis acht Tage aufrechterhalten werden, er kann individuell etwas variieren. Es ist bekannt, zu diesem Zweck einen Druckverband anzulegen, der ein Kissen enthält, das grösser als das Hauttransplantat ist und dieses auf seiner ganzen Fläche gleichmässig auf den wunden Körperteil aufdrückt (Ferris Smith: Plastic and Reconstructive Surgery, W. B. Saunders Co., 1950, Seiten 26 und 27). Es lässt sich jedoch auch bei Anwendung grösster Sorgfalt nicht immer vermeiden, dass die auf dem Transplantat aufliegende Fläche des Kissens durch den Druckverband Falten wirft, insbesondere wenn die Haut auf stark gekrümmte Körperpartien verpflanzt werden muss und die Transplantate gross sind. Diese Falten bilden sich über dem übertragenen Hautstück ab und sind auch nach dem völligen Abheilen sichtbar.

Aus der US-A-1 320 105 ist ein Bruchband bekannt, das einen aufblasbaren Gummisack aufweist, der auf der einen Seite auf einer Körperpartie aufliegt und auf der anderen Seite mit einem steifen Oberteil versehen ist. Über dieses steife Oberteil laufen Bänder, mit denen das Bruchband am Körper befestigt wird. Dieses bekannte Bruchband verfügt jedoch über keinerlei Mittel, um einen definierten Druck im Gummisack 1 einzustellen, ausserdem gestattet es keine Beobachtung des unterhalb des Bruchbandes liegenden Körperbereiches.

Aus der DE-A-2 810 737 ist ein Apparat zur Behandlung von inneren Verletzungen oder Entzündungen des menschlichen Körpers bekannt, bei dem an einem Gürtel eine Platte befestigt ist, die ihrerseits einen balgartigen Beutel trägt, mit dem ein Tampon auf eine Körperoberfläche gedrückt werden kann. Der Balg ist aufblasbar. Der bekannte Apparat dient jedoch weder zum Andrücken von Hauttransplantaten noch sind Mittel zum genauen Einstellen oder Konstanthalten des Luftdruckes im Balg vorgesehen, noch eignet sich der bekannte Apparat zum Betrachten des Körperbereiches, auf dem der Apparat aufliegt.

In der DE-A-2 124 803 ist ein fester Umhüllungsverband zur Stillegung gebrochener Glieder und anderer Körperteile beschrieben, bei dem ein fester, prothesenartiger Verband im Bereiche der inneren Oberfläche mit Luftkammern ausgestattet ist, so dass beispielsweise ein gebrochenes Bein gesamthaft umhüllt und gehalten werden kann. Auch diese Anordnung dient nicht zum Andrücken von Hauttransplantaten, ausserdem fehlt es auch bei ihr an Mitteln zum Einstellen und Konstanthalten des Luftdruckes, und schliesslich ist auch keine Betrachtung des Körperbereiches möglich.

Schliesslich zeigt die DE-U-7 806 873 noch eine Vorrichtung zum Ausspülen von Wunden, bei der eine durchsichtige Hülle über eine offene Wunde gesetzt wird und über Zu- und Ableitungen eine Spülflüssigkeit durch die Hülle leitbar ist. Zwar gestattet diese Vorrichtung die Betrachtung der gespülten Wunde, es handelt sich jedoch um eine reine Therapieeinrichtung und nicht um eine solche der Nachsorge, ausserdem ist weder eine für Verbandszwecke geeignete starre Platte vorgesehen noch herrscht im Inneren der Hülle dieser Vorrichtung ein definierter Überdruck, wie er zum Andrücken von Hauttransplantaten erforderlich ist, es herrscht vielmehr bestimmungsgemäss ein Unterdruck.

Der Erfindung liegt demgegenüber die Aufgabe zugrunde, einen Druckverband für ein Hauttransplantat zu entwickeln, der die Nachteile des eingangs genannten Verbandes, wie man ihn bei Hauttransplantationen verwendet, vermeidet.

Diese Aufgabe wird erfindungsgemäss dadurch gelöst, dass der Verband auf der dem Körper abgewandten Seite des Kissens eine steife Platte aufweist, dass die Platte aus einem durchsichtigen Werkstoff besteht und dass das Kissen auf der der Platte abgewandten Seite eine durchsichtige Folie aufweist.

Die Erfindung hat damit den Vorteil, dass beim Anlegen des Druckverbandes der von dem Verband ausgeübte Druck sich über die Platte gleichmässig auf das Kissen verteilt. Die steife Platte verhindert ausserdem, dass sich das Kissen, das in der Regel mit Luft oder Wasser gefüllt wird, einkerbt, wenn die erste Windung des Druckverbandes über das Kissen gelegt wird, wodurch sich Abschnitte der auf dem Transplantat aufliegenden Fläche des Luftkissens relativ zu der Körperoberlfläche etwas verschieben können und dadurch eine Faltenbildung begünstigen, auch wenn die auf dem Körper aufliegende Folie des Kissens elastisch ist.

Die Platte besteht dabei aus einem durchsichtigen Werkstoff. Dies ermöglicht, durch die Platte hindurch die auf der Körperoberfläche aufliegende Fläche des Kissens während des Anlegens des Druckverbandes daraufhin zu überprüfen, ob sich Falten bilden. Weiterhin ist auch die auf der Körperoberfläche aufliegende Fläche des Kissens aus einem durchsichtigen Werkstoff, beispielsweise aus einer durchsichtigen Folie, so dass das Transplantat bei angelegtem Druckverband, in dem selbstverständlich ein entprechendes Fenster ausgespart sein muss, beobachtet werden kann.

Die Grösse der Platte soll so gewählt sein, dass sie die Funktion der Druckverteilung und das Verhindern des Einkerbens erfüllt. Bei einer Ausführungsform der Erfindung ist die Platte mindestens nahezu so gross wie das Hauttransplantat. Dadurch wird erreicht, dass eine Faltenbildung im Bereich des Transplantates mit sehr grosser Wahrscheinlichkeit verhindert werden kann.

Die Druckverteilung und die Verhinderung von Faltenbildung ist bei der durchsichtigen Platte deswegen besonders wichtig, weil die über die Platte gewickelte Binde — wie erwähnt — ein Fenster freilassen muss, so dass keine gleichmässige Wickelung des Verbandes mit der Binde möglich ist.

Es ist zweckmässig, gemäss einer Ausführungsform der Erfindung die Platte der Form der Körperoberfläche anzupassen. Beispielsweise kann zum Anlegen eines Druckverbandes an einer Extremität die Platte die Form einer der Körperform angepassten Schale aufweisen, oder kann eine Körperform der Platte dadurch angenähert werden, dass mehrere Platten aneinandergefügt werden, zum Beispiel kann der ganze Umfang eines Beinabschnittes mit Hilfe von mehreren aneinander angelegten Platten abgedeckt werden, wobei sich das Kissen zwischen diesen Platten und der Beinoberfläche befindet.

In der Zeichnung ist eine Aufführungsform der Erfindung dargestellt.

Fig. 1 zeigt eine Seitenansicht einer Ausführungsform des erfindungsgemässen Druckverbandes,

Fig. 2 zeigt eine andere Ausführungsform mit sehr stark gekrümmter Platte in Ansicht und

Fig. 3 einen Schnitt durch die Ausführungsform nach Fig. 2.

Bei der in der Zeichnung dargestellten Ausführungsform der Erfindung weist das Kissen zwei Folienzuschnitte 1 und 2 aus Latex oder einem geeigneten, vorzugsweise elastischen Kunststoff auf, die an ihren Rändern durch eine Naht 3 luftdicht miteinander verbunden sind. An derjenigen Seite des Kissens, die beim Anlegen des Druckverbandes von der zu behandelnden Körperoberfläche abgewandt ist, ist auf die Folie 1 eine steife Platte 4 aufgechweisst oder aufgeklebt oder auf andere Weise in ihrer Lage fixiert, die die Form der Oberfläche der Körperpartie 5 aufweist, auf die das Hautstück verpflanzt wird. Diese Kunststoffplatte ist so steif und so gross, dass sie den von dem Druckverband ausgeübten Druck gleichmässig über die Oberfläche des Kissens verteilt und dadurch eine Faltenbildung der Folie 2 auf der Unterseite des Kissens verhindert. Vorzugsweise ist die Platte 4 gleich gross oder grösser als das Hauttransplantat.

Die Platte 4 besteht aus einem durchsichtigen Werkstoff und die Folie 2 ist ebenfalls durchsichtig, so dass, sofern der Druckverband an der entsprechenden Stelle ebenfalls ein Fenster aufweist, das Hauttransplantat durch dieses Fenster, die durchsichtige Platte 4 und die durchsichtige Folie 2 hindurch beobachtet werden kann.

In der Nähe des Randes des Kissens ist in die Folie 1 ein Ventil 6 eingeschweisst, das zum Füllen des Kissens mit Wasser oder Luft dient. Das Ventil 6 ist so ausgebildet, dass an dieses Vorrichtungen zum Erzeugen, Aufrechterhalten und/oder Überwachen des Druckes im Kissen angeschlossen werden können.

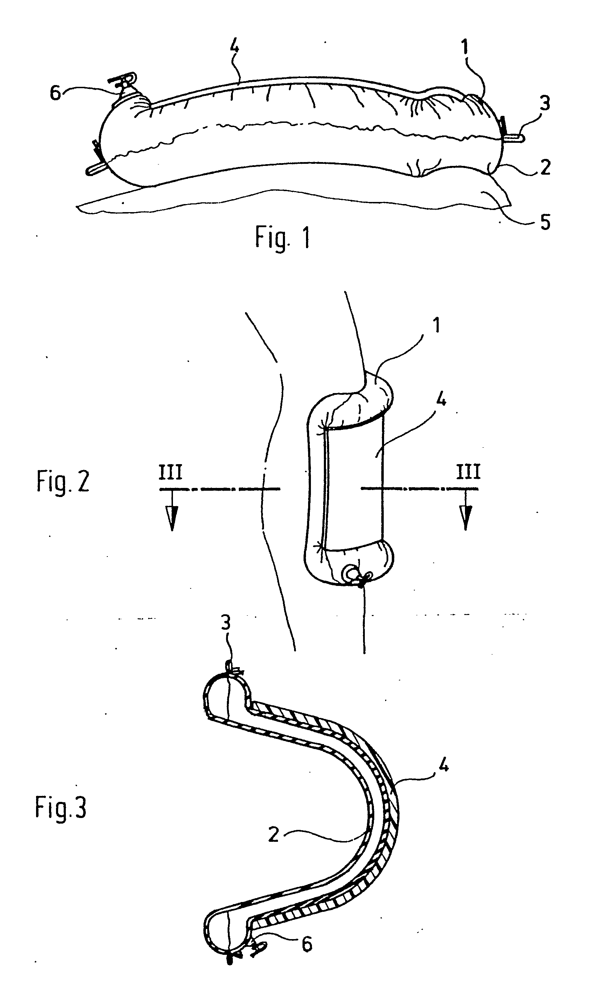

Die Fig. 2 und 3 zeigen ein Anwendungsbeispiel des erfindungsgemässen Druckverbandes, der bei dieser Ausführungsform so angepasst ist, dass er bei der Transplantation eines Hautabschnittes auf ein Bein im Bereich des Schienbeines verwendet werden kann. Die Platte 4 ist entsprechend dieser Körperoberfläche im Bereich des Schienbeines stark gekrümmt, wie der in Fig. 3 dargestellte Querschnitt zeigt.

Das Kissen kann mit Hilfe einer Binde oder einem elastischen Strumpf oder aber auf andere Weise über dem Transplantat befestigt sein, z.B. auch festgeklebt sein. Auch Können die beiden Folien 1 und 2 eine unterschiedliche Grösse aufweisen, z.B. die auf der Haut aufliegende Folie 2 über die Naht 3 hinaus verlängert sein und zum Befestigen des Kissens dienen. Auch können mehrere gewölbte und/oder ebene Platten auf dem Kissen befestigt sein, die dann durch die Folie des Kissens gelenkig miteinander verbunden sind. Die Platte kann aus einem thermoplastischen Kunststoff bestehen und vor dem Anlegen des Verbandes, vor oder nach dem Befestigen auf dem Kissen erwärmt und in die Körperform verformt werden.

## Patentansprüche

1. Ein Kissen enthaltender Druckverband zum Andrücken von in Körpern (5) eingesetzten Hauttransplantaten, bei dem das Kissen mit einem Druckmedium gefüllt ist, Mittel zum Überwachen des Druckes im Kissen anschliessbar sind und über dem Kissen ein den Druck ausübender Teil des Verbandes angeordnet ist, zum Beispiel eine Binde gewickelt ist, dadurch gekennzeichnet, dass der Verband auf der dem Körper (5) abgewandten Seite des Kissens eine steife Platte (4) aufweist, dass die Platte (4) aus einem durchsichtigen Werkstoff besteht und dass das Kissen auf der der Platte (4) abgewandten Seite eine durchsichtige Folie (2) aufweist.

2. Verband nach Anspruch 1, dadurch gekennzeichnet, dass die Platte (4) zumindest nahezu so gross ist wie das Hauttransplantat.

3. Verband nach Anspruch 1 oder 2, dadurch gekennzeichnet, dass die Platte (4) der Form der Körperoberfläche (5) angepasst ist, auf der das Hauttransplantat aufgelegt ist.

4. Verband nach Anspruch 3, dadurch gekennzeichnet, dass zur Anlegung eines Durckverbandes an einer Extremität die Platte (4) die Form einer Schale aufweist.

5. Verband nach einem der vorhergehenden Ansprüche, dadurch gekennzeichnet, dass die Platte (4) auf dem Kissen fixiert ist.

6. Verband nach einem der vorhergehenden Ansprüche, dadurch gekennzeichnet, dass mehrere Platten auf dem Kissen befestigt sind.

## Claims

1. A pressure pad for applying a skin graft to be inserted into a living body (5), wherein a cushion is filled with a pressure medium, means for controlling the pressure within the cushion may be connected to the cushion and a part of the pad, exerting pressure is positioned above the cushion, e.g. in the form of a wound bandage, characterized in that the pad comprises a stiff plate (4) on the side of the cushion opposite to the body (5), said plate (4) being made of a transparent material and that the cushion is provided with a transparent foil (2) on the side opposite said plate (4).

2. A dressing according to claim 1, characterized in that the plate (4) is at least approximately as large as the skin graft.

3. A dressing according to claim 1 or 2, characterized in that the plate (4) is shaped to conform to

## 0 035 584

the shape of the body surface (5) onto which the skin section has been grafted.

4. A dressing according to claim 3, characterized in that a pressure dressing for use on an extremity is provided with a plate (4) which is dish-shaped.

5. Dressing according to any of the preceding claims, characterized in that said plate (4) is fixedly secured to the cushion.

6. Dressing according to any of the preceding claims, characterized in that a plurality of plates are secured to the cushion.

### Revendications

1. Pansement de compression contenant un coussin destiné à maintenir par compression un implant de transfert de peau mis en place dans des corps (5), pansement dans lequel le coussin est rempli avec un fluide de pression, des moyens pouvant être raccordés pour surveiller la pression dans le coussin, une partie du pansement, par exemple un bandage, étant enroulée par-dessus le coussin pour exercer la pression, pansement caractérisé en ce qu'il présente, sur le côté du coussin opposé à la surface appuyée sur le corps (5), une plaque rigide (4), en une matière transparente, le coussin étant pourvu, sur sa face éloignée de la plaque (4) d'une feuille (2) de matière transparente.

2. Pansement suivant la revendication 1, caractérisé en ce que la plaque (4) est d'une grandeur au moins égale à celle du transplant de peau.

3. Pansement suivant l'une des revendications 1 ou 2, caractérisé en ce que la plaque (4) est adaptée à la forme de la surface du corps (5) sur laquelle le transplant de peau est appliqué.

4. Pansement suivant la revendication 3, caractérisé en ce que, pour l'application d'un pansement avec pression, la plaque (4) présente, à une extrémité, la forme d'une coquille.

5. Pansement suivant l'une quelconque des revendications 1 à 4, caractérisé en ce que la plaque (4) est fixée à demeure sur le coussin.

6. Pansement suivant l'une qualconque des revendications 1 à 5, caractérisé en ce que plusieurs plaques sont fixées sur le coussin.

Fig. 1

Fig. 2

Fig.3